# EUROPEAN PATENT SPECIFICATION

(11) **EP 3 873 246 B1**
(45) Date of publication and mention of the grant of the patent: **19.08.2026**
(21) Application number: 19798366.1
(22) Date of filing: 31.10.2019
(51) Int. Cl.: A24B 15/167, A24F 40/10

(54) **AEROSOLISED FORMULATION**
AEROSOLFÄHIGE FORMULIERUNG
FORMULATION EN AÉROSOL

(30) Priority: 01.11.2018 GB 201817868
(43) Date of publication of application: 08.09.2021
(73) Proprietor: Nicoventures Trading Limited, London WC2R 3LA (GB)
(72) Inventor: CABOT, Ross, London WC2R 3LA (GB)
(74) Representative: D Young & Co LLP
(86) International application number: PCT/GB2019/053093
(87) International publication number: WO 2020/089638

(56) References cited:
- EP-A1- 3 574 902
- WO-A1-2014/116974
- US-A- 4 945 928
- US-A1- 2016 198 759

## Description

### FIELD OF THE INVENTION

The present disclosure relates to an aerosolised formulation, a method of forming the same, a device for forming the same and processes and uses of the same.

### BACKGROUND TO THE INVENTION

Electronic aerosol provision systems such as e-cigarettes generally contain a reservoir of liquid which is to be vaporised, typically containing a flavour or an active agent such as nicotine. When a user inhales on the device, a heater is activated to vaporise a small amount of liquid, which is therefore inhaled by the user.

The use of e-cigarettes in the UK has grown rapidly, and it has been estimated that there are now over a million people using them in the UK.

One challenge faced in providing such systems is to provide from the aerosol provision device a aerosol to be inhaled which provides consumers with an acceptable experience. Some consumers may prefer an e-cigarette that generates an aerosol that closely 'mimics' smoke inhaled from a tobacco product such as a cigarette. Aerosols from e-cigarettes and smoke from tobacco products such as cigarettes provides to the user a complex chain of flavour in the mouth, and if nicotine is present, nicotine absorption in the mouth and throat, followed by nicotine absorption in the lungs. Document US2016/198759A1 discloses an improved non-nicotine fluid for e-cigarette devices. These various aspects are described by users in terms of flavour, intensity/quality, impact, irritation/smoothness and reward. Flavour contributes to a number of these factors, and is strongly associated with flavour in the mouth and the provision of desirable taste and smell, whether mimicking the taste and smell of a tobacco product or providing alternative flavours. Reliably providing a particular taste and smell is made more difficult by the volatile and thermally sensitive nature of some flavours. Heating of flavour components in e-cigarettes may result in some flavours being degraded. This has a number of disadvantages. Flavours present in the liquid may be lost resulting in a diminished flavour experience for the user or the need to include in the liquid excess flavour at additional cost. Furthermore if e-liquid contains multiple flavours and only some of these multiple flavours are degraded, this can adversely affect the balance of the taste and smell. Furthermore, degraded flavours may have an undesirable or "off taste". Each of these factors, and their balance, can strongly contribute to consumer acceptability of an e-cigarette. Providing means to optimise the overall vaping experience is therefore desirable to e-cigarette manufacturers.

A further challenge facing such systems is the continued demand for harm reduction. Harm from cigarette and e-cigarette devices primarily comes from toxicants. Therefore, there is a desire to reduce the potential for the formation of toxicants.

### SUMMARY OF THE INVENTION

In one aspect there is provided an aerosolised formulation comprising
(i) water in an amount of at least 50 wt.% based on the aerosolised formulation; and
(ii) at least one active agent;
wherein the aerosol has the following particle size distribution: D50 of from 2 to 6µm and wherein the aerosol has a D10 of at least 0.5µm.

In one aspect there is provided a process for forming an aerosol, the process comprising aerosolising an aerosolisable formulation comprising
(i) water in an amount of at least 50 wt.% based on the aerosolisable formulation; and
(ii) at least one active agent; to provide an aerosol having the following particle size distribution: D50 of from 3 to 6µm and D10 of at least 0.5µm.

In one aspect there is provided an electronic aerosol provision system comprising:
(a) an aerosoliser for aerosolising formulation for inhalation by a user of the electronic aerosol provision system;
(b) a power supply comprising a cell or battery for supplying power to the aerosoliser
(c) aerosolisable formulation comprising
   (i) water in an amount of at least 50 wt.% based on the aerosolisable formulation; and
   (ii) at least one active agent;
wherein the aerosoliser provides an aerosol having the following particle size distribution: D50 of from 2 to 6µm and D10 of at least 0.5µm.

In one aspect there is provided a process for improving delivery of an aerosolised formulation to the lungs, the process comprising the steps of
(a) providing an aerosolisable material comprising (i) water in an amount of at least 50 wt.% based on the aerosolisable material and (ii) at least one active agent;
(b) aerosolising the material aerosolisable material to provide an aerosol having the following particle size distribution: D50 of from 2 to 6µm and D10 of at least 0.5µm.

### DETAILED DESCRIPTION

As discussed herein in one aspect there is provided an aerosolised formulation comprising
(i) water in an amount of at least 50 wt.% based on the aerosolised formulation; and
(ii) at least one active agent;
wherein the aerosol has the following particle size distribution: D50 of from 2 to 6µm and wherein the aerosol has a D10 of at least 0.5µm.

We have found that an advantageous system may be provided in which an aerosolised formulation containing a high content of water and an active agent is formed from an aerosolisable formulation. In particular, we have identified that a water-based system may be provided in which aerosolised formulation is formed from an aerosolisable formulation at a low temperature. This is in contrast to 'traditional' e-cigarettes which use a heater, typically applied to liquids based on glycerol and propylene glycol to form an aerosolised formulation. The provision of such a system and the avoidance of heating may address problems of the prior art relating to the formation of toxicants. More specifically, we have identified a water-based system which, by careful selection of the particle size distribution of the aerosol, delivers water droplets containing the active agent into desirable areas of the lungs, such as the deep lung. In particular, the aerosol has a D50 particle size distribution of from 2 to 6µm. We have identified that in water-based aerosols, the droplets have a tendency to evaporate and decrease in size when inhaled by the user. Therefore the specific selection of particle size described herein addresses the reduction of size through evaporation and still delivers the active agent into the desired area of the lungs. These problems and the choice made in the present invention is in contrast to the prior liquids based on glycerol and propylene glycol in which the liquid droplets tend to adhere to moisture within the lungs and thereby increase in size when inhaled.

For ease of reference, these and further aspects of the present invention are now discussed under appropriate section headings. However, the teachings under each section are not necessarily limited to each particular section.

### Water

As discussed herein the aerosolised formulation comprises water in an amount of at least 50 wt.% based on the aerosolised formulation. In one aspect water is present in an amount of at least 55 wt.% based on the aerosolised formulation. In one aspect water is present in an amount of at least 60 wt.% based on the aerosolised formulation. In one aspect water is present in an amount of at least 65 wt.% based on the aerosolised formulation. In one aspect water is present in an amount of at least 70 wt.% based on the aerosolised formulation. In one aspect water is present in an amount of at least 75 wt.% based on the aerosolised formulation. In one aspect water is present in an amount of at least 80 wt.% based on the aerosolised formulation. In one aspect water is present in an amount of at least 85 wt.% based on the aerosolised formulation. In one aspect water is present in an amount of at least 90 wt.% based on the aerosolised formulation. In one aspect water is present in an amount of at least 95 wt.% based on the aerosolised formulation. In one aspect water is present in an amount of at least 99 wt.% based on the aerosolised formulation.

In one aspect water is present in an amount of from 50 to 99 wt.% based on the aerosolised formulation. In one aspect water is present in an amount of from 55 to 99 wt.% based on the aerosolised formulation. In one aspect water is present in an amount of from 60 to 99 wt.% based on the aerosolised formulation. In one aspect water is present in an amount of from 65 to 99 wt.% based on the aerosolised formulation. In one aspect water is present in an amount of from 70 to 99 wt.% based on the aerosolised formulation. In one aspect water is present in an amount of from 75 to 99 wt.% based on the aerosolised formulation. In one aspect water is present in an amount of from 80 to 99 wt.% based on the aerosolised formulation. In one aspect water is present in an amount of from 85 to 99 wt.% based on the aerosolised formulation. In one aspect water is present in an amount of from 90 to 99 wt.% based on the aerosolised formulation. In one aspect water is present in an amount of from 95 to 99 wt.% based on the aerosolised formulation.

As discussed herein the aerosolisable formulation comprises water in an amount of at least 50 wt.% based on the aerosolisable formulation. In one aspect water is present in an amount of at least 55 wt.% based on the aerosolisable formulation. In one aspect water is present in an amount of at least 60 wt.% based on the aerosolisable formulation. In one aspect water is present in an amount of at least 65 wt.% based on the aerosolisable formulation. In one aspect water is present in an amount of at least 70 wt.% based on the aerosolisable formulation. In one aspect water is present in an amount of at least 75 wt.% based on the aerosolisable formulation. In one aspect water is present in an amount of at least 80 wt.% based on the aerosolisable formulation. In one aspect water is present in an amount of at least 85 wt.% based on the aerosolisable formulation. In one aspect water is present in an amount of at least 90 wt.% based on the aerosolisable formulation. In one aspect water is present in an amount of at least 95 wt.% based on the aerosolisable formulation. In one aspect water is present in an amount of at least 99 wt.% based on the aerosolisable formulation.

In one aspect water is present in an amount of from 50 to 99 wt.% based on the aerosolisable formulation. In one aspect water is present in an amount of from 55 to 99 wt.% based on the aerosolisable formulation. In one aspect water is present in an amount of from 60 to 99 wt.% based on the aerosolisable formulation. In one aspect water is present in an amount of from 65 to 99 wt.% based on the aerosolisable formulation. In one aspect water is present in an amount of from 70 to 99 wt.% based on the aerosolisable formulation. In one aspect water is present in an amount of from 75 to 99 wt.% based on the aerosolisable formulation. In one aspect water is present in an amount of from 80 to 99 wt.% based on the aerosolisable formulation. In one aspect water is present in an amount of from 85 to 99 wt.% based on the aerosolisable formulation. In one aspect water is present in an amount of from 90 to 99 wt.% based on the aerosolisable formulation. In one aspect water is present in an amount of from 95 to 99 wt.% based on the aerosolisable formulation.

As discussed herein the use of water allows for the replacement of some or all of the glycerol, propylene glycol, 1,3-propane diol and mixtures thereof typically used in e-cigarettes. In one aspect the aerosolisable formulation contains glycerol, propylene glycol, 1,3-propane diol and mixtures thereof in a combined amount of no greater than 10 wt.% based on the aerosolisable formulation or based on the aerosolised formulation. In one aspect the aerosolisable formulation contains glycerol, propylene glycol, 1,3-propane diol and mixtures thereof in a combined amount of no greater than 8 wt.% based on the aerosolisable formulation or based on the aerosolised formulation. In one aspect the aerosolisable formulation contains glycerol, propylene glycol, 1,3-propane diol and mixtures thereof in a combined amount of no greater than 5 wt.% based on the aerosolisable formulation or based on the aerosolised formulation. In one aspect the aerosolisable formulation contains glycerol, propylene glycol, 1,3-propane diol and mixtures thereof in a combined amount of no greater than 2 wt.% based on the aerosolisable formulation or based on the aerosolised formulation. In one aspect the aerosolisable formulation contains glycerol, propylene glycol, 1,3-propane diol and mixtures thereof in a combined amount of no greater than 1 wt.% based on the aerosolisable formulation or based on the aerosolised formulation. In one aspect the aerosolisable formulation contains glycerol, propylene glycol, 1,3-propane diol and mixtures thereof in a combined amount of no greater than 0.5 wt.% based on the aerosolisable formulation or based on the aerosolised formulation. In one aspect the aerosolisable formulation contains glycerol, propylene glycol, 1,3-propane diol and mixtures thereof in a combined amount of no greater than 0.2 wt.% based on the aerosolisable formulation or based on the aerosolised formulation. In one aspect the aerosolisable formulation contains glycerol, propylene glycol, 1,3-propane diol and mixtures thereof in a combined amount of no greater than 0.1 wt.% based on the aerosolisable formulation or based on the aerosolised formulation. In one aspect the aerosolisable formulation contains glycerol, propylene glycol, 1,3-propane diol and mixtures thereof in a combined amount of no greater than 0.01 wt.% based on the aerosolisable formulation or based on the aerosolised formulation. In one aspect the aerosolisable formulation contains no glycerol, propylene glycol, 1,3-propane diol and mixtures thereof.

In one aspect the aerosolisable formulation contains glycerol, propylene glycol, and mixtures thereof in a combined amount of no greater than 10 wt.% based on the aerosolisable formulation or based on the aerosolised formulation. In one aspect the aerosolisable formulation contains glycerol, propylene glycol, and mixtures thereof in a combined amount of no greater than 8 wt.% based on the aerosolisable formulation or based on the aerosolised formulation. In one aspect the aerosolisable formulation contains glycerol, propylene glycol, and mixtures thereof in a combined amount of no greater than 5 wt.% based on the aerosolisable formulation or based on the aerosolised formulation. In one aspect the aerosolisable formulation contains glycerol, propylene glycol, and mixtures thereof in a combined amount of no greater than 2 wt.% based on the aerosolisable formulation or based on the aerosolised formulation. In one aspect the aerosolisable formulation contains glycerol, propylene glycol, and mixtures thereof in a combined amount of no greater than 1 wt.% based on the aerosolisable formulation or based on the aerosolised formulation. In one aspect the aerosolisable formulation contains glycerol, propylene glycol, and mixtures thereof in a combined amount of no greater than 0.5 wt.% based on the aerosolisable formulation or based on the aerosolised formulation. In one aspect the aerosolisable formulation contains glycerol, propylene glycol, and mixtures thereof in a combined amount of no greater than 0.2 wt.% based on the aerosolisable formulation or based on the aerosolised formulation. In one aspect the aerosolisable formulation contains glycerol, propylene glycol, and mixtures thereof in a combined amount of no greater than 0.1 wt.% based on the aerosolisable formulation or based on the aerosolised formulation. In one aspect the aerosolisable formulation contains glycerol, propylene glycol, and mixtures thereof in a combined amount of no greater than 0.01 wt.% based on the aerosolisable formulation or based on the aerosolised formulation. In one aspect the aerosolisable formulation contains no glycerol, propylene glycol, and mixtures thereof.

In one aspect the aerosolisable formulation contains glycerol in an amount of no greater than 10 wt.% based on the aerosolisable formulation or based on the aerosolised formulation. In one aspect the aerosolisable formulation contains glycerol in an amount of no greater than 8 wt.% based on the aerosolisable formulation or based on the aerosolised formulation. In one aspect the aerosolisable formulation contains glycerol in an amount of no greater than 5 wt.% based on the aerosolisable formulation or based on the aerosolised formulation. In one aspect the aerosolisable formulation contains glycerol in an amount of no greater than 2 wt.% based on the aerosolisable formulation or based on the aerosolised formulation. In one aspect the aerosolisable formulation contains glycerol in an amount of no greater than 1 wt.% based on the aerosolisable formulation or based on the aerosolised formulation. In one aspect the aerosolisable formulation contains glycerol in an amount of no greater than 0.5 wt.% based on the aerosolisable formulation or based on the aerosolised formulation. In one aspect the aerosolisable formulation contains glycerol in an amount of no greater than 0.2 wt.% based on the aerosolisable formulation or based on the aerosolised formulation. In one aspect the aerosolisable formulation contains glycerol in an amount of no greater than 0.1 wt.% based on the aerosolisable formulation or based on the aerosolised formulation. In one aspect the aerosolisable formulation contains glycerol in an amount of no greater than 0.01 wt.% based on the aerosolisable formulation or based on the aerosolised formulation. In one aspect the aerosolisable formulation contains no glycerol.

In one aspect the aerosolisable formulation contains propylene glycol in an amount of no greater than 10 wt.% based on the aerosolisable formulation or based on the aerosolised formulation. In one aspect the aerosolisable formulation contains propylene glycol in an amount of no greater than 8 wt.% based on the aerosolisable formulation or based on the aerosolised formulation. In one aspect the aerosolisable formulation contains propylene glycol in an amount of no greater than 5 wt.% based on the aerosolisable formulation or based on the aerosolised formulation. In one aspect the aerosolisable formulation contains propylene glycol in an amount of no greater than 2 wt.% based on the aerosolisable formulation or based on the aerosolised formulation. In one aspect the aerosolisable formulation contains propylene glycol in an amount of no greater than 1 wt.% based on the aerosolisable formulation or based on the aerosolised formulation. In one aspect the aerosolisable formulation contains propylene glycol in an amount of no greater than 0.5 wt.% based on the aerosolisable formulation or based on the aerosolised formulation. In one aspect the aerosolisable formulation contains propylene glycol in an amount of no greater than 0.2 wt.% based on the aerosolisable formulation or based on the aerosolised formulation. In one aspect the aerosolisable formulation contains propylene glycol in an amount of no greater than 0.1 wt.% based on the aerosolisable formulation or based on the aerosolised formulation. In one aspect the aerosolisable formulation contains propylene glycol in an amount of no greater than 0.01 wt.% based on the aerosolisable formulation or based on the aerosolised formulation. In one aspect the aerosolisable formulation contains no propylene glycol.

### Active Agent

The aerosolisable formulation and the aerosolised formulation further comprise an active agent. By "active agent" it is meant an agent which has a biological effect, such as sensorial and/or physiological effect, on a subject when the aerosol is inhaled. In one aspect by "active agent" it is meant an agent which has a physiological effect on a subject when the aerosol is inhaled. In some cases, the active may be a flavour. The active agent may for example be selected from nutraceuticals, nootropics, psychoactives. The active substance may be naturally occurring or synthetically obtained. The active agent may comprise for example nicotine, caffeine, taurine, theine, vitamins such as B6 or B12 or C, melatonin, cannabinoids, or constituents, derivatives, or combinations thereof. The active agent may comprise one or more constituents, derivatives or extracts of tobacco, cannabis or another botanical. In some embodiments, the active agent comprises caffeine, melatonin or vitamin B12. The one or more active agents may be selected from nicotine, botanicals, and mixtures thereof. The one or more active agents may be of synthetic or natural origin. The active could be an extract from a botanical, such as from a plant in the tobacco family. In one aspect the active agent is at least nicotine. In one aspect the active agent consists of nicotine and/or salts thereof.

As noted herein, the active agent may comprise one or more constituents, derivatives or extracts of cannabis, such as one or more cannabinoids or terpenes. Cannabinoids are a class of natural or synthetic chemical compounds which act on cannabinoid receptors (i.e., CB1 and CB2) in cells that repress neurotransmitter release in the brain. Cannabinoids may be naturally occurring (phytocannabinoids) from plants such as cannabis, from animals (endocannabinoids), or artificially manufactured (synthetic cannabinoids). Cannabis species express at least 85 different phytocannabinoids, and are divided into subclasses, including cannabigerols, cannabichromenes, cannabidiols, tetrahydrocannabinols, cannabinols and cannabinodiols, and other cannabinoids. Cannabinoids found in cannabis include, without limitation: cannabigerol (CBG), cannabichromene (CBC), cannabidiol (CBD), tetrahydrocannabinol (THC), cannabinol (CBN), cannabinodiol (CBDL), cannabicyclol (CBL), cannabivarin (CBV), tetrahydrocannabivarin (THCV), cannabidivarin (CBDV), cannabichromevarin (CBCV), cannabigerovarin (CBGV), cannabigerol monomethyl ether (CBGM), cannabinerolic acid, cannabidiolic acid (CBDA), Cannabinol propyl variant (CBNV), cannabitriol (CBO), tetrahydrocannabmolic acid (THCA), and tetrahydrocannabivarinic acid (THCV A).

In some embodiments, the active agent comprises one or more constituents, derivatives or extracts of cannabis.

As noted herein, the active agent may comprise or be derived from one or more botanicals or constituents, derivatives or extracts thereof. As used herein, the term "botanical" includes any material derived from plants including, but not limited to, extracts, leaves, bark, fibres, stems, roots, seeds, flowers, fruits, pollen, husk, shells or the like. Alternatively, the material may comprise an active compound naturally existing in a botanical, obtained synthetically. The material may be in the form of liquid, gas, solid, powder, dust, crushed particles, granules, pellets, shreds, strips, sheets, or the like. Example botanicals are tobacco, eucalyptus, star anise, hemp, cocoa, cannabis, fennel, lemongrass, peppermint, spearmint, rooibos, chamomile, flax, ginger, ginkgo biloba, hazel, hibiscus, laurel, licorice (liquorice), matcha, mate, orange skin, papaya, rose, sage, tea such as green tea or black tea, thyme, clove, cinnamon, coffee, aniseed (anise), basil, bay leaves, cardamom, coriander, cumin, nutmeg, oregano, paprika, rosemary, saffron, lavender, lemon peel, mint, juniper, elderflower, vanilla, wintergreen, beefsteak plant, curcuma, turmeric, sandalwood, cilantro, bergamot, orange blossom, myrtle, cassis, valerian, pimento, mace, damien, marjoram, olive, lemon balm, lemon basil, chive, carvi, verbena, tarragon, geranium, mulberry, ginseng, theanine, theacrine, maca, ashwagandha, damiana, guarana, chlorophyll, baobab or any combination thereof. The mint may be chosen from the following mint varieties: Mentha Arventis, Mentha c.v.,Mentha niliaca, Mentha piperita, Mentha piperita citrata c.v.,Mentha piperita c.v, Mentha spicata crispa, Mentha cardifolia, Memtha longifolia, Mentha suaveolens variegata, Mentha pulegium, Mentha spicata c.v. and Mentha suaveolens.

In some embodiments, the active agent comprises or derived from one or more botanicals or constituents, derivatives or extracts thereof and the botanical is tobacco.

In some embodiments, the active agent comprises or derived from one or more botanicals or constituents, derivatives or extracts thereof and the botanical is selected from eucalyptus, star anise, cocoa and hemp.

In some embodiments, the active agent comprises or derived from one or more botanicals or constituents, derivatives or extracts thereof and the botanical is selected from rooibos and fennel.

In one aspect the one or more active agents are present in a combined amount (such as nicotine is present in an amount) of no greater than 6 wt% based on the aerosolisable formulation or based on the aerosolised formulation. In one aspect the one or more active agents are present in a combined amount (such as nicotine is present in an amount) of from 0.01 to 6 wt% based on the aerosolisable formulation or based on the aerosolised formulation. In one aspect the one or more active agents are present in a combined amount (such as nicotine is present in an amount) of from 0.02 to 6 wt% based on the aerosolisable formulation or based on the aerosolised formulation. In one aspect the one or more active agents are present in a combined amount (such as nicotine is present in an amount) of from 0.05 to 6 wt% based on the aerosolisable formulation or based on the aerosolised formulation. In one aspect the one or more active agents are present in a combined amount (such as nicotine is present in an amount) of from 0.08 to 6 wt% based on the aerosolisable formulation or based on the aerosolised formulation. In one aspect the one or more active agents are present in a combined amount (such as nicotine is present in an amount) of from 0.01 to 5 wt% based on the aerosolisable formulation or based on the aerosolised formulation. In one aspect the one or more active agents are present in a combined amount (such as nicotine is present in an amount) of from 0.02 to 5 wt% based on the aerosolisable formulation or based on the aerosolised formulation. In one aspect the one or more active agents are present in a combined amount (such as nicotine is present in an amount) of from 0.05 to 5 wt% based on the aerosolisable formulation or based on the aerosolised formulation. In one aspect the one or more active agents are present in a combined amount (such as nicotine is present in an amount) of from 0.08 to 5 wt% based on the aerosolisable formulation or based on the aerosolised formulation. In one aspect the one or more active agents are present in a combined amount (such as nicotine is present in an amount) of no greater than 4 wt% based on the aerosolisable formulation or based on the aerosolised formulation. In one aspect the one or more active agents are present in a combined amount (such as nicotine is present in an amount) of from 0.01 to 4 wt% based on the aerosolisable formulation or based on the aerosolised formulation. In one aspect the one or more active agents are present in a combined amount (such as nicotine is present in an amount) of from 0.02 to 4 wt% based on the aerosolisable formulation or based on the aerosolised formulation. In one aspect the one or more active agents are present in a combined amount (such as nicotine is present in an amount) of from 0.05 to 4 wt% based on the aerosolisable formulation or based on the aerosolised formulation. In one aspect the one or more active agents are present in a combined amount (such as nicotine is present in an amount) of from 0.08 to 4 wt% based on the aerosolisable formulation or based on the aerosolised formulation. In one aspect the one or more active agents are present in a combined amount (such as nicotine is present in an amount) of no greater than 3 wt% based on the aerosolisable formulation or based on the aerosolised formulation. In one aspect the one or more active agents are present in a combined amount (such as nicotine is present in an amount) of from 0.01 to 3 wt% based on the aerosolisable formulation or based on the aerosolised formulation. In one aspect the one or more active agents are present in a combined amount (such as nicotine is present in an amount) of from 0.02 to 3 wt% based on the aerosolisable formulation or based on the aerosolised formulation. In one aspect the one or more active agents are present in a combined amount (such as nicotine is present in an amount) of from 0.05 to 3 wt% based on the aerosolisable formulation or based on the aerosolised formulation. In one aspect the one or more active agents are present in a combined amount (such as nicotine is present in an amount) of from 0.08 to 3 wt% based on the aerosolisable formulation or based on the aerosolised formulation. In one aspect the one or more active agents are present in a combined amount (such as nicotine is present in an amount) of no greater than 2 wt% based on the aerosolisable formulation or based on the aerosolised formulation. In one aspect the one or more active agents are present in a combined amount (such as nicotine is present in an amount) of from 0.01 to 2 wt% based on the aerosolisable formulation or based on the aerosolised formulation. In one aspect the one or more active agents are present in a combined amount (such as nicotine is present in an amount) of from 0.02 to 2 wt% based on the aerosolisable formulation or based on the aerosolised formulation. In one aspect the one or more active agents are present in a combined amount (such as nicotine is present in an amount) of from 0.05 to 2 wt% based on the aerosolisable formulation or based on the aerosolised formulation. In one aspect the one or more active agents are present in a combined amount (such as nicotine is present in an amount) of from 0.08 to 2 wt% based on the aerosolisable formulation or based on the aerosolised formulation. In one aspect the one or more active agents are present in a combined amount (such as nicotine is present in an amount) of no greater than 1 wt% based on the aerosolisable formulation or based on the aerosolised formulation. In one aspect the one or more active agents are present in a combined amount (such as nicotine is present in an amount) of from 0.01 to 1 wt% based on the aerosolisable formulation or based on the aerosolised formulation. In one aspect the one or more active agents are present in a combined amount (such as nicotine is present in an amount) of from 0.02 to 1 wt% based on the aerosolisable formulation or based on the aerosolised formulation. In one aspect the one or more active agents are present in a combined amount (such as nicotine is present in an amount) of from 0.05 to 1 wt% based on the aerosolisable formulation or based on the aerosolised formulation. In one aspect the one or more active agents are present in a combined amount (such as nicotine is present in an amount) of from 0.08 to 1 wt% based on the aerosolisable formulation or based on the aerosolised formulation. In one aspect the one or more active agents are present in a combined amount (such as nicotine is present in an amount) of from 0.1 to 1 wt% based on the aerosolisable formulation or based on the aerosolised formulation. In one aspect the one or more active agents are present in a combined amount (such as nicotine is present in an amount) of no greater than 0.6 wt% based on the aerosolisable formulation or based on the aerosolised formulation. In one aspect the one or more active agents are present in a combined amount (such as nicotine is present in an amount) of from 0.01 to 0.6 wt% based on the aerosolisable formulation or based on the aerosolised formulation. In one aspect the one or more active agents are present in a combined amount (such as nicotine is present in an amount) of from 0.02 to 0.6 wt% based on the aerosolisable formulation or based on the aerosolised formulation. In one aspect the one or more active agents are present in a combined amount (such as nicotine is present in an amount) of from 0.05 to 0.6 wt% based on the aerosolisable formulation or based on the aerosolised formulation. In one aspect the one or more active agents are present in a combined amount (such as nicotine is present in an amount) of from 0.08 to 0.6 wt% based on the aerosolisable formulation or based on the aerosolised formulation. In one aspect the one or more active agents are present in a combined amount (such as nicotine is present in an amount) of from 0.1 to 0.6 wt% based on the aerosolisable formulation or based on the aerosolised formulation. In one aspect the one or more active agents are present in a combined amount (such as nicotine is present in an amount) of no greater than 0.5 wt% based on the aerosolisable formulation or based on the aerosolised formulation. In one aspect the one or more active agents are present in a combined amount (such as nicotine is present in an amount) of from 0.01 to 0.5 wt% based on the aerosolisable formulation or based on the aerosolised formulation. In one aspect the one or more active agents are present in a combined amount (such as nicotine is present in an amount) of from 0.02 to 0.5 wt% based on the aerosolisable formulation or based on the aerosolised formulation. In one aspect the one or more active agents are present in a combined amount (such as nicotine is present in an amount) of from 0.05 to 0.5 wt% based on the aerosolisable formulation or based on the aerosolised formulation. In one aspect the one or more active agents are present in a combined amount (such as nicotine is present in an amount) of from 0.08 to 0.5 wt% based on the aerosolisable formulation or based on the aerosolised formulation. In one aspect the one or more active agents are present in a combined amount (such as nicotine is present in an amount) of no greater than 0.2 wt% based on the aerosolisable formulation or based on the aerosolised formulation. In one aspect the one or more active agents are present in a combined amount (such as nicotine is present in an amount) of from 0.01 to 0.2 wt% based on the aerosolisable formulation or based on the aerosolised formulation. In one aspect the one or more active agents are present in a combined amount (such as nicotine is present in an amount) of from 0.02 to 0.2 wt% based on the aerosolisable formulation or based on the aerosolised formulation. In one aspect the one or more active agents are present in a combined amount (such as nicotine is present in an amount) of from 0.05 to 0.2 wt% based on the aerosolisable formulation or based on the aerosolised formulation. In one aspect the one or more active agents are present in a combined amount (such as nicotine is present in an amount) of from 0.08 to 0.2 wt% based on the aerosolisable formulation or based on the aerosolised formulation. In one aspect the one or more active agents are present in a combined amount (such as nicotine is present in an amount) of no greater than 0.1 wt% based on the aerosolisable formulation or based on the aerosolised formulation. In one aspect the one or more active agents are present in a combined amount (such as nicotine is present in an amount) of from 0.01 to 0.1 wt% based on the aerosolisable formulation or based on the aerosolised formulation. In one aspect the one or more active agents are present in a combined amount (such as nicotine is present in an amount) of from 0.02 to 0.1 wt% based on the aerosolisable formulation or based on the aerosolised formulation. In one aspect the one or more active agents are present in a combined amount (such as nicotine is present in an amount) of from 0.05 to 0.1 wt% based on the aerosolisable formulation or based on the aerosolised formulation. In one aspect the one or more active agents are present in a combined amount (such as nicotine is present in an amount) of from 0.08 to 0.1 wt% based on the aerosolisable formulation or based on the aerosolised formulation.

### Particle Size Distribution

The aerosol of the aerosolised formulation has the following particle size distribution: D50 of from 2 to 6µm and D10 of at least 0.5µm.

References in the present specification to particle size distribution, D50, D10 or D90 refer to values measured in accordance with British and European Pharmacopoeia, 2.9.31 Particle Size Analysis By Laser Light Diffraction (see BRITISH PHARMACOPOEIA COMMISSION. (2014), British Pharmacopoeia. London, England: Stationery Office and COUNCIL OF EUROPE. (2013). European Pharmacopoeia. Strasbourg, France: Council of Europe). The terms D50, Dv50 and Dx50 are interchangeable. The terms D10, Dv10 and Dx10 are interchangeable. The terms D90, Dv90 and Dx90 are interchangeable.

In one aspect the aerosol has a D50 of from 2.5 to 6µm. In one aspect the aerosol has a D50 of from 3 to 6µm. In one aspect the aerosol has a D50 of from 3.5 to 6µm. In one aspect the aerosol has a D50 of from 4 to 6µm. In one aspect the aerosol has a D50 of from 4.5 to 6µm. In one aspect the aerosol has a D50 of from 5 to 6µm. In one aspect the aerosol has a D50 of from 2.5 to 5.5µm. In one aspect the aerosol has a D50 of from 3 to 5.5µm. In one aspect the aerosol has a D50 of from 3.5 to 5.5µm. In one aspect the aerosol has a D50 of from 4 to 5.5µm. In one aspect the aerosol has a D50 of from 4.5 to 5.5µm. In one aspect the aerosol has a D50 of from 5 to 5.5µm. In one aspect the aerosol has a D50 of from 4 to 5.5µm. In one aspect the aerosol has a D50 of from 4 to 5µm. In one aspect the aerosol has a D50 of from 4 to 4.5µm. In one aspect the aerosol has a D50 of from 2.5 to 5µm. In one aspect the aerosol has a D50 of from 2.5 to 4.5µm.

According to the invention, the aerosol has a D10 of at least 0.5µm. In one aspect the aerosol has a D10 of at least 1µm. In one aspect the aerosol has a D10 of at least 1.5µm. In one aspect the aerosol has a D10 of at least 2µm.

In one aspect the aerosol has a D90 of no greater than 15µm. In one aspect the aerosol has a D90 of no greater than 12µm. In one aspect the aerosol has a D90 of no greater than 10µm.

In one aspect D50 is measured after exclusion of particles having a particle size of less than 1µm. In one aspect D10 is measured after exclusion of particles having a particle size of less than 1µm. In one aspect D90 is measured after exclusion of particles having a particle size of less than 1µm.

In one aspect D50 is measured after exclusion of particles having a particle size of less than 1µm and the aerosol has a D50 of from 2 to 6µm. In one aspect D50 is measured after exclusion of particles having a particle size of less than 1µm and the aerosol has a D50 of from 2.5 to 6µm. In one aspect D50 is measured after exclusion of particles having a particle size of less than 1µm and the aerosol has a D50 of from 3 to 6µm. In one aspect D50 is measured after exclusion of particles having a particle size of less than 1µm and the aerosol has a D50 of from 3.5 to 6µm. In one aspect D50 is measured after exclusion of particles having a particle size of less than 1µm and the aerosol has a D50 of from 4 to 6µm. In one aspect D50 is measured after exclusion of particles having a particle size of less than 1µm and the aerosol has a D50 of from 4.5 to 6µm. In one aspect D50 is measured after exclusion of particles having a particle size of less than 1µm and the aerosol has a D50 of from 5 to 6µm. In one aspect D50 is measured after exclusion of particles having a particle size of less than 1µm and the aerosol has a D50 of from 2.5 to 5.5µm. In one aspect D50 is measured after exclusion of particles having a particle size of less than 1µm and the aerosol has a D50 of from 3 to 5.5µm. In one aspect D50 is measured after exclusion of particles having a particle size of less than 1µm and the aerosol has a D50 of from 3.5 to 5.5µm. In one aspect D50 is measured after exclusion of particles having a particle size of less than 1µm and the aerosol has a D50 of from 4 to 5.5µm. In one aspect D50 is measured after exclusion of particles having a particle size of less than 1µm and the aerosol has a D50 of from 4.5 to 5.5µm. In one aspect D50 is measured after exclusion of particles having a particle size of less than 1µm and the aerosol has a D50 of from 5 to 5.5µm. In one aspect D50 is measured after exclusion of particles having a particle size of less than 1µm and the aerosol has a D50 of from 4 to 5.5µm. In one aspect D50 is measured after exclusion of particles having a particle size of less than 1µm and the aerosol has a D50 of from 4 to 5µm. In one aspect D50 is measured after exclusion of particles having a particle size of less than 1µm and the aerosol has a D50 of from 4 to 4.5µm. In one aspect D50 is measured after exclusion of particles having a particle size of less than 1µm and the aerosol has a D50 of from 2.5 to 5µm. In one aspect D50 is measured after exclusion of particles having a particle size of less than 1µm and the aerosol has a D50 of from 2.5 to 4.5µm.

In one aspect D10 is measured after exclusion of particles having a particle size of less than 1µm and the aerosol has a D10 of at least 0.5µm. In one aspect D10 is measured after exclusion of particles having a particle size of less than 1µm and the aerosol has a D10 of at least 1µm. In one aspect D10 is measured after exclusion of particles having a particle size of less than 1µm and the aerosol has a D10 of at least 1.5µm. In one aspect D10 is measured after exclusion of particles having a particle size of less than 1µm and the aerosol has a D10 of at least 2µm.

In one aspect D90 is measured after exclusion of particles having a particle size of less than 1µm and the aerosol has a D90 of no greater than 15µm. In one aspect D90 is measured after exclusion of particles having a particle size of less than 1µm and the aerosol has a D90 of no greater than 12µm. In one aspect D90 is measured after exclusion of particles having a particle size of less than 1µm and the aerosol has a D90 of no greater than 10µm.

In one aspect D50 is measured after exclusion of particles having a particle size of greater than 30µm. In one aspect D10 is measured after exclusion of particles having a particle size of greater than 30µm. In one aspect D90 is measured after exclusion of particles having a particle size of greater than 30µm.

In one aspect D50 is measured after exclusion of particles having a particle size of greater than 30µm and the aerosol has a D50 of from 2 to 6µm. In one aspect D50 is measured after exclusion of particles having a particle size of greater than 30µm and the aerosol has a D50 of from 2.5 to 6µm. In one aspect D50 is measured after exclusion of particles having a particle size of greater than 30µm and the aerosol has a D50 of from 3 to 6µm. In one aspect D50 is measured after exclusion of particles having a particle size of greater than 30µm and the aerosol has a D50 of from 3.5 to 6µm. In one aspect D50 is measured after exclusion of particles having a particle size of greater than 30µm and the aerosol has a D50 of from 4 to 6µm. In one aspect D50 is measured after exclusion of particles having a particle size of greater than 30µm and the aerosol has a D50 of from 4.5 to 6µm. In one aspect D50 is measured after exclusion of particles having a particle size of greater than 30µm and the aerosol has a D50 of from 5 to 6µm. In one aspect D50 is measured after exclusion of particles having a particle size of greater than 30µm and the aerosol has a D50 of from 2.5 to 5.5µm. In one aspect D50 is measured after exclusion of particles having a particle size of greater than 30µm and the aerosol has a D50 of from 3 to 5.5µm. In one aspect D50 is measured after exclusion of particles having a particle size of greater than 30µm and the aerosol has a D50 of from 3.5 to 5.5µm. In one aspect D50 is measured after exclusion of particles having a particle size of greater than 30µm and the aerosol has a D50 of from 4 to 5.5µm. In one aspect D50 is measured after exclusion of particles having a particle size of greater than 30µm and the aerosol has a D50 of from 4.5 to 5.5µm. In one aspect D50 is measured after exclusion of particles having a particle size of greater than 30µm and the aerosol has a D50 of from 5 to 5.5µm. In one aspect D50 is measured after exclusion of particles having a particle size of greater than 30µm and the aerosol has a D50 of from 4 to 5.5µm. In one aspect D50 is measured after exclusion of particles having a particle size of greater than 30µm and the aerosol has a D50 of from 4 to 5µm. In one aspect D50 is measured after exclusion of particles having a particle size of greater than 30µm and the aerosol has a D50 of from 4 to 4.5µm. In one aspect D50 is measured after exclusion of particles having a particle size of greater than 30µm and the aerosol has a D50 of from 2.5 to 5µm. In one aspect D50 is measured after exclusion of particles having a particle size of greater than 30µm and the aerosol has a D50 of from 2.5 to 4.5µm.

In one aspect D10 is measured after exclusion of particles having a particle size of greater than 30µm and the aerosol has a D10 of at least 0.5µm. In one aspect D10 is measured after exclusion of particles having a particle size of greater than 30µm and the aerosol has a D10 of at least 1µm. In one aspect D10 is measured after exclusion of particles having a particle size of greater than 30µm and the aerosol has a D10 of at least 1.5µm. In one aspect D10 is measured after exclusion of particles having a particle size of greater than 30µm and the aerosol has a D10 of at least 2µm.

In one aspect D90 is measured after exclusion of particles having a particle size of greater than 30µm and the aerosol has a D90 of no greater than 15µm. In one aspect D90 is measured after exclusion of particles having a particle size of greater than 30µm and the aerosol has a D90 of no greater than 12µm. In one aspect D90 is measured after exclusion of particles having a particle size of greater than 30µm and the aerosol has a D90 of no greater than 10µm. In one aspect D90 is measured after exclusion of particles having a particle size of greater than 30µm and the aerosol has a D90 is from 10 to 30µm.

### Acid

As discussed above, the aerosolisable formulation and the aerosolised formulation of the present invention may contain one or more further components. These components may be selected depending on the nature of the formulation. In one aspect, the aerosolisable formulation and the aerosolised formulation further comprises an acid

In one aspect the acid is an organic acid. In one aspect the acid is a carboxylic acid. In one aspect the acid is an organic carboxylic acid.

In one aspect the acid is selected from the group consisting of acetic acid, lactic acid, formic acid, citric acid, benzoic acid, pyruvic acid, levulinic acid, succinic acid, tartaric acid, sorbic acid, propionic acid, phenylacetic acid, and mixtures thereof. In one aspect the acid is selected from the group consisting of citric acid, benzoic acid, levulinic acid, lactic acid, sorbic acid, and mixtures thereof. In one aspect the acid is selected from the group consisting of citric acid, benzoic acid, levulinic acid, and mixtures thereof. In one aspect the acid is at least citric acid. In one aspect the acid consists of citric acid.

In one aspect the acid is selected from acids having a pka of from 2 to 5. In one aspect the acid is a weak acid. In one aspect the acid is a weak organic acid.

In one aspect the acid has a solubility in water of at least 5g/L at 20 °C. In one aspect the acid has a solubility in water of at least 10g/L at 20 °C. In one aspect the acid has a solubility in water of at least 20g/L at 20 °C. In one aspect the acid has a solubility in water of at least 50g/L at 20 °C. In one aspect the acid has a solubility in water of at least 100g/L at 20 °C. In one aspect the acid has a solubility in water of at least 200g/L at 20 °C. In one aspect the acid has a solubility in water of at least 300g/L at 20 °C. In one aspect the acid has a solubility in water of at least 400g/L at 20 °C. In one aspect the acid has a solubility in water of at least 500g/L at 20 °C. In one aspect the acid has a solubility in water of at least 600g/L at 20 °C. In one aspect the acid has a solubility in water of at least 700g/L at 20 °C. In one aspect the acid has a solubility in water of at least 800g/L at 20 °C. In one aspect the acid has a solubility in water of at least 900g/L at 20 °C. In one aspect the acid has a solubility in water of at least 1000g/L at 20 °C. In one aspect the acid has a solubility in water of at least 1100g/L at 20 °C.

The molar ratio of acid to active agent (such as nicotine) may be selected as desired. In one aspect the molar ratio of acid to active agent (such as nicotine) is from 5:1 to 1:5. In one aspect the molar ratio of acid to active agent (such as nicotine) is from 4:1 to 1:4. In one aspect the molar ratio of acid to active agent (such as nicotine) is from 3:1 to 1:3. In one aspect the molar ratio of acid to active agent (such as nicotine) is from 2:1 to 1:2. In one aspect the molar ratio of acid to active agent (such as nicotine) is from 1.5:1 to 1:1.5. In one aspect the molar ratio of acid to active agent (such as nicotine) is from 1.2:1 to 1:1.2. In one aspect the molar ratio of acid to active agent (such as nicotine) is from 5:1 to 1:1. In one aspect the molar ratio of acid to active agent (such as nicotine) is from 4:1 to 1:1. In one aspect the molar ratio of acid to active agent (such as nicotine) is from 3:1 to 1:1. In one aspect the molar ratio of acid to active agent (such as nicotine) is from 2:1 to 1:1. In one aspect the molar ratio of acid to active agent (such as nicotine) is from 1.5:1 to 1:1. In one aspect the molar ratio of acid to active agent (such as nicotine) is from 1.2:1 to 1:1.

In one aspect the total content of acid present in the formulation is no greater than 5 mole equivalents based on the active agent (such as nicotine). In one aspect the total content of acid present in the formulation is no greater than 4 mole equivalents based on the active agent (such as nicotine). In one aspect the total content of acid present in the formulation is no greater than 3 mole equivalents based on the active agent (such as nicotine). In one aspect the total content of acid present in the formulation is no greater than 2 mole equivalents based on the active agent (such as nicotine). In one aspect the total content of acid present in the formulation is no greater than 1 mole equivalents based on the active agent (such as nicotine).

In one aspect the total content of acid present in the formulation is no less than 0.01 mole equivalents based on the active agent (such as nicotine). In one aspect the total content of acid present in the formulation is no less than 0.05 mole equivalents based on the active agent (such as nicotine). In one aspect the total content of acid present in the formulation is no less than 0.1 mole equivalents based on the active agent (such as nicotine). In one aspect the total content of acid present in the formulation is no less than 0.2 mole equivalents based on the active agent (such as nicotine). In one aspect the total content of acid present in the formulation is no less than 0.3 mole equivalents based on the active agent (such as nicotine). In one aspect the total content of acid present in the formulation is no less than 0.4 mole equivalents based on the active agent (such as nicotine). In one aspect the total content of acid present in the formulation is no less than 0.5 mole equivalents based on the active agent (such as nicotine). In one aspect the total content of acid present in the formulation is no less than 0.7 mole equivalents based on the active agent (such as nicotine).

In one aspect the acid is present in an amount of no greater than 6 wt% based on the aerosolisable formulation. In one aspect the acid is present in an amount of from 0.01 to 6 wt% based on the aerosolisable formulation. In one aspect the acid is present in an amount of from 0.02 to 6 wt% based on the aerosolisable formulation. In one aspect the acid is present in an amount of from 0.05 to 6 wt% based on the aerosolisable formulation. In one aspect the acid is present in an amount of from 0.08 to 6 wt% based on the aerosolisable formulation. In one aspect the acid is present in an amount of from 0.01 to 5 wt% based on the aerosolisable formulation. In one aspect the acid is present in an amount of from 0.02 to 5 wt% based on the aerosolisable formulation. In one aspect the acid is present in an amount of from 0.05 to 5 wt% based on the aerosolisable formulation. In one aspect the acid is present in an amount of from 0.08 to 5 wt% based on the aerosolisable formulation. In one aspect the acid is present in an amount of no greater than 4 wt% based on the aerosolisable formulation. In one aspect the acid is present in an amount of from 0.01 to 4 wt% based on the aerosolisable formulation. In one aspect the acid is present in an amount of from 0.02 to 4 wt% based on the aerosolisable formulation. In one aspect the acid is present in an amount of from 0.05 to 4 wt% based on the aerosolisable formulation. In one aspect the acid is present in an amount of from 0.08 to 4 wt% based on the aerosolisable formulation. In one aspect the acid is present in an amount of no greater than 3 wt% based on the aerosolisable formulation. In one aspect the acid is present in an amount of from 0.01 to 3 wt% based on the aerosolisable formulation. In one aspect the acid is present in an amount of from 0.02 to 3 wt% based on the aerosolisable formulation. In one aspect the acid is present in an amount of from 0.05 to 3 wt% based on the aerosolisable formulation. In one aspect the acid is present in an amount of from 0.08 to 3 wt% based on the aerosolisable formulation. In one aspect the acid is present in an amount of no greater than 2 wt% based on the aerosolisable formulation. In one aspect the acid is present in an amount of from 0.01 to 2 wt% based on the aerosolisable formulation. In one aspect the acid is present in an amount of from 0.02 to 2 wt% based on the aerosolisable formulation. In one aspect the acid is present in an amount of from 0.05 to 2 wt% based on the aerosolisable formulation. In one aspect the acid is present in an amount of from 0.08 to 2 wt% based on the aerosolisable formulation. In one aspect the acid is present in an amount of no greater than 1 wt% based on the aerosolisable formulation. In one aspect the acid is present in an amount of from 0.01 to 1 wt% based on the aerosolisable formulation. In one aspect the acid is present in an amount of from 0.02 to 1 wt% based on the aerosolisable formulation. In one aspect the acid is present in an amount of from 0.05 to 1 wt% based on the aerosolisable formulation. In one aspect the acid is present in an amount of from 0.08 to 1 wt% based on the aerosolisable formulation. In one aspect the acid is present in an amount of from 0.1 to 1 wt% based on the aerosolisable formulation. In one aspect the acid is present in an amount of no greater than 0.6 wt% based on the aerosolisable formulation. In one aspect the acid is present in an amount of from 0.01 to 0.6 wt% based on the aerosolisable formulation. In one aspect the acid is present in an amount of from 0.02 to 0.6 wt% based on the aerosolisable formulation. In one aspect the acid is present in an amount of from 0.05 to 0.6 wt% based on the aerosolisable formulation. In one aspect the acid is present in an amount of from 0.08 to 0.6 wt% based on the aerosolisable formulation. In one aspect the acid is present in an amount of from 0.1 to 0.6 wt% based on the aerosolisable formulation. In one aspect the acid is present in an amount of no greater than 0.5 wt% based on the aerosolisable formulation. In one aspect the acid is present in an amount of from 0.01 to 0.5 wt% based on the aerosolisable formulation. In one aspect the acid is present in an amount of from 0.02 to 0.5 wt% based on the aerosolisable formulation. In one aspect the acid is present in an amount of from 0.05 to 0.5 wt% based on the aerosolisable formulation. In one aspect the acid is present in an amount of from 0.08 to 0.5 wt% based on the aerosolisable formulation. In one aspect the acid is present in an amount of no greater than 0.2 wt% based on the aerosolisable formulation. In one aspect the acid is present in an amount of from 0.01 to 0.2 wt% based on the aerosolisable formulation. In one aspect the acid is present in an amount of from 0.02 to 0.2 wt% based on the aerosolisable formulation. In one aspect the acid is present in an amount of from 0.05 to 0.2 wt% based on the aerosolisable formulation. In one aspect the acid is present in an amount of from 0.08 to 0.2 wt% based on the aerosolisable formulation. In one aspect the acid is present in an amount of no greater than 0.1 wt% based on the aerosolisable formulation. In one aspect the acid is present in an amount of from 0.01 to 0.1 wt% based on the aerosolisable formulation. In one aspect the acid is present in an amount of from 0.02 to 0.1 wt% based on the aerosolisable formulation. In one aspect the acid is present in an amount of from 0.05 to 0.1 wt% based on the aerosolisable formulation. In one aspect the acid is present in an amount of from 0.08 to 0.1 wt% based on the aerosolisable formulation.

The amount of acid and the solubility of the acid may be selected such that a given amount of the acid will dissolve in the water. In one aspect at 20 °C at least 20% of the acid dissolves in the water. In one aspect at 25 °C at least 20% of the acid dissolves in the water. In one aspect at 30 °C at least 20% of the acid dissolves in the water. In one aspect at 20 °C at least 35% of the acid dissolves in the water. In one aspect at 20 °C at least 40% of the acid dissolves in the water. In one aspect at 20 °C at least 45% of the acid dissolves in the water. In one aspect at 20 °C at least 50% of the acid dissolves in the water. In one aspect at 20 °C at least 55% of the acid dissolves in the water.

As is understood by one skilled in the art, nicotine may exist in unprotonated form, monoprotonated form or diprotonated form. The structures of each of these forms are given below.

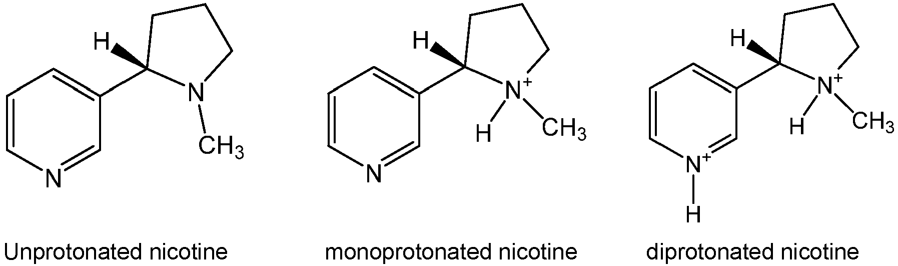

Reference in the specification to protonated form means both monoprotonated nicotine and diprotonated nicotine. Reference in the specification to amounts in the protonated form means the combined amount of monoprotonated nicotine and diprotonated nicotine. Furthermore, when reference is made to a fully protonated formulation it will be understood that at any one time there may be very minor amounts of unprotonated nicotine present, e.g. less than 1% unprotonated.

The formulation may comprise nicotine in protonated form. The formulation may comprise nicotine in unprotonated form. In one aspect the formulation comprises nicotine in unprotonated form and nicotine in monoprotonated form. In one aspect the formulation comprises nicotine in unprotonated form and nicotine in diprotonated form. In one aspect the formulation comprises nicotine in unprotonated form, nicotine in monoprotonated form and nicotine in diprotonated form.

In one aspect at least 5wt% of the nicotine present in the formulation is in protonated form. In one aspect at least 10wt% of the nicotine present in the formulation is in protonated form. In one aspect at least 15wt% of the nicotine present in the formulation is in protonated form. In one aspect at least 20wt% of the nicotine present in the formulation is in protonated form. In one aspect at least 25wt% of the nicotine present in the formulation is in protonated form. In one aspect at least 30wt% of the nicotine present in the formulation is in protonated form. In one aspect at least 35wt% of the nicotine present in the formulation is in protonated form. In one aspect at least 40wt% of the nicotine present in the formulation is in protonated form. In one aspect at least 45wt% of the nicotine present in the formulation is in protonated form. In one aspect at least 50wt% of the nicotine present in the formulation is in protonated form. In one aspect at least 55wt% of the nicotine present in the formulation is in protonated form. In one aspect at least 60wt% of the nicotine present in the formulation is in protonated form. In one aspect at least 65wt% of the nicotine present in the formulation is in protonated form.

In one aspect at least 70wt% of the nicotine present in the formulation is in protonated form. In one aspect at least 75wt% of the nicotine present in the formulation is in protonated form. In one aspect at least 80wt% of the nicotine present in the formulation is in protonated form. In one aspect at least 85wt% of the nicotine present in the formulation is in protonated form. In one aspect at least 90wt% of the nicotine present in the formulation is in protonated form. In one aspect at least 95wt% of the nicotine present in the formulation is in protonated form. In one aspect at least 99wt% of the nicotine present in the formulation is in protonated form. In one aspect at least 99.9wt% of the nicotine present in the formulation is in protonated form.

In one aspect from 50 to 95 wt% of the nicotine present in the formulation is in protonated form. In one aspect from 55 to 95 wt% of the nicotine present in the formulation is in protonated form. In one aspect from 60 to 95 wt% of the nicotine present in the formulation is in protonated form. In one aspect from 65 to 95 wt% of the nicotine present in the formulation is in protonated form. In one aspect from 70 to 95 wt% of the nicotine present in the formulation is in protonated form. In one aspect from 75 to 95 wt% of the nicotine present in the formulation is in protonated form. In one aspect from 80 to 95 wt% of the nicotine present in the formulation is in protonated form. In one aspect from 85 to 95 wt% of the nicotine present in the formulation is in protonated form. In one aspect from 90 to 95 wt% of the nicotine present in the formulation is in protonated form.

The relevant amounts of nicotine which are present in the formulation in protonated form are specified herein. These amounts may be readily calculated by one skilled in the art. Nicotine, 3-(1-methylpyrrolidin-2-yl) pyridine, is a diprotic base with pKa of 3.12 for the pyridine ring and 8.02 for the pyrrolidine ring It can exist in pH-dependent protonated (mono- and di-) and non-protonated (free base) forms which have different bioavailability.

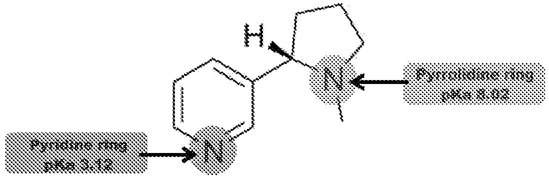

The distribution of protonated and non-protonated nicotine will vary at various pH increments.

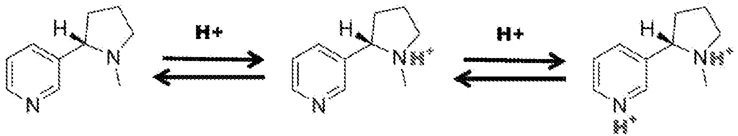

The fraction of non-protonated nicotine will be predominant at high pH levels whilst a decrease in the pH will see an increase of the fraction of protonated nicotine (mono- or di-depending on the pH). If the relative fraction of protonated nicotine and the total amount of nicotine in the sample are known, the absolute amount of protonated nicotine can be calculated.

The relative fraction of protonated nicotine in formulation can be calculated by using the Henderson-Hasselbalch equation, which describes the pH as a derivation of the acid dissociation constant equation, and it is extensively employed in chemical and biological systems. Consider the following equilibrium:

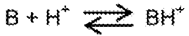

The Henderson-Hasselbalch equation for this equilibrium is: $pH = pKa + log \frac{\left[B\right]}{\left[BH+\right]}$

Where [B] is the amount of non-protonated nicotine (i.e. free base), [BH+] the amount of protonated nicotine (i.e. conjugate acid) and pKa is the reference pKa value for the pyrrolidine ring nitrogen of nicotine (pKa=8.02). The relative fraction of protonated nicotine can be derived from the alpha value of the non-protonated nicotine calculated from the Henderson-Hasselbalch equation as: $% protonated nicotine = 100 - \left\{\frac{\frac{\left[B\right]}{\left[BH+\right]}}{\left\{1+\frac{\left[B\right]}{\left[BH+\right]}\right\}}∗100\right\}$

Determination of pKa values of nicotine formulations was carried out using the basic approach described in "Spectroscopic investigations into the acid-base properties of nicotine at different temperatures", Peter M. Clayton, Carl A. Vas, Tam T. T. Bui, Alex F. Drake and Kevin McAdam, .Anal. Methods, 2013,5, 81-88.

### Flavour

The aerosolisable formulation and the aerosolised formulation of the present invention may contain one or more further components. These components may be selected depending on the nature of the formulation. The aerosolisable formulation may comprise one or more flavours or flavouring components. As used herein, the terms "flavour" and "flavourant" refer to materials which, where local regulations permit, may be used to create a desired taste or aroma in a product for adult consumers. They may include extracts (e.g. liquorice, hydrangea, Japanese white bark magnolia leaf, chamomile, fenugreek, clove, menthol, Japanese mint, aniseed, cinnamon, herb, wintergreen, cherry, berry, peach, apple, Drambuie, bourbon, scotch, whiskey, spearmint, peppermint, lavender, cardamom, celery, cascarilla, nutmeg, sandalwood, bergamot, geranium, honey essence, rose oil, vanilla, lemon oil, orange oil, cassia, caraway, cognac, jasmine, ylang-ylang, sage, fennel, piment, ginger, anise, coriander, coffee, or a mint oil from any species of the genus Mentha), flavour enhancers, bitterness receptor site blockers, sensorial receptor site activators or stimulators, sugars and/or sugar substitutes (e.g., sucralose, acesulfame potassium, aspartame, saccharine, cyclamates, lactose, sucrose, glucose, fructose, sorbitol, or mannitol), and other additives such as charcoal, chlorophyll, minerals, botanicals, or breath freshening agents. They may be imitation, synthetic or natural ingredients or blends thereof. They may be in any suitable form, for example, oil, liquid, or powder. The one or more flavours may be selected from the group consisting of (4-(para-)methoxyphenyl)-2-butanone, vanillin, γ-undecalactone, menthone, 5-propenyl guaethol, menthol, para-mentha-8-thiol-3-one and mixtures thereof. In one aspect the flavour is at least menthol.

If present, the one or more flavours may be present in any suitable amount. In one aspect the one or more flavours are present in a total amount of no greater than 10 wt.% based on the aerosolisable formulation. In one aspect the one or more flavours are present in a total amount of no greater than 7 wt.% based on the aerosolisable formulation. In one aspect the one or more flavours are present in a total amount of no greater than 5 wt.% based on the aerosolisable formulation. In one aspect the one or more flavours are present in a total amount of no greater than 4 wt.% based on the aerosolisable formulation. In one aspect the one or more flavours are present in a total amount of no greater than 3 wt.% based on the aerosolisable formulation. In one aspect the one or more flavours are present in a total amount of no greater than 2wt.% based on the aerosolisable formulation. In one aspect the one or more flavours are present in a total amount of no greater than 1wt.% based on the aerosolisable formulation.

In one aspect the one or more flavours are present in a total amount of from 0.01 to 5wt.% based on the aerosolisable formulation. In one aspect the one or more flavours are present in a total amount of from 0.01 to 4wt.% based on the aerosolisable formulation. In one aspect the one or more flavours are present in a total amount of from 0.01 to 3wt.% based on the aerosolisable formulation. In one aspect the one or more flavours are present in a total amount of from 0.01 to 2wt.% based on the aerosolisable formulation. In one aspect the one or more flavours are present in a total amount of from 0.01 to 1wt.% based on the aerosolisable formulation. In one aspect the one or more flavours are present in a total amount of from 0.01 to 0.5wt.% based on the aerosolisable formulation.

In one aspect the one or more flavours are present in a total amount of no greater than 10 wt.% based on the aerosolised formulation. In one aspect the one or more flavours are present in a total amount of no greater than 7 wt.% based on the aerosolised formulation. In one aspect the one or more flavours are present in a total amount of no greater than 5 wt.% based on the aerosolised formulation. In one aspect the one or more flavours are present in a total amount of no greater than 4 wt.% based on the aerosolised formulation. In one aspect the one or more flavours are present in a total amount of no greater than 3 wt.% based on the aerosolised formulation. In one aspect the one or more flavours are present in a total amount of no greater than 2wt.% based on the aerosolised formulation. In one aspect the one or more flavours are present in a total amount of no greater than 1wt.% based on the aerosolised formulation.

In one aspect the one or more flavours are present in a total amount of from 0.01 to 5wt.% based on the aerosolised formulation. In one aspect the one or more flavours are present in a total amount of from 0.01 to 4wt.% based on the aerosolised formulation. In one aspect the one or more flavours are present in a total amount of from 0.01 to 3wt.% based on the aerosolised formulation. In one aspect the one or more flavours are present in a total amount of from 0.01 to 2wt.% based on the aerosolised formulation. In one aspect the one or more flavours are present in a total amount of from 0.01 to 1wt.% based on the aerosolised formulation. In one aspect the one or more flavours are present in a total amount of from 0.01 to 0.5wt.% based on the aerosolised formulation.

### Formulation

One or more cyclodextrins may or may not be present in any suitable amount in the aerosolisable formulation. In one aspect the one or more cyclodextrins are present in a total amount of no greater than 12 wt.% based on the aerosolisable formulation. In one aspect the one or more cyclodextrins are present in a total amount of no greater than 10 wt.% based on the aerosolisable formulation. In one aspect the one or more cyclodextrins are present in a total amount of no greater than 9 wt.% based on the aerosolisable formulation. In one aspect the one or more cyclodextrins are present in a total amount of no greater than 8 wt.% based on the aerosolisable formulation. In one aspect the one or more cyclodextrins are present in a total amount of no greater than 7 wt.% based on the aerosolisable formulation. In one aspect the one or more cyclodextrins are present in a total amount of no greater than 6 wt.% based on the aerosolisable formulation. In one aspect the one or more cyclodextrins are present in a total amount of no greater than 5 wt.% based on the aerosolisable formulation. In one aspect the one or more cyclodextrins are present in a total amount of no greater than 4 wt.% based on the aerosolisable formulation. In one aspect the one or more cyclodextrins are present in a total amount of no greater than 3 wt.% based on the aerosolisable formulation. In one aspect the one or more cyclodextrins are present in a total amount of no greater than 2 wt.% based on the aerosolisable formulation. In one aspect the one or more cyclodextrins are present in a total amount of no greater than 1 wt.% based on the aerosolisable formulation. In one aspect the one or more cyclodextrins are present in a total amount of no greater than 0.5 wt.% based on the aerosolisable formulation. In one aspect the one or more cyclodextrins are present in a total amount of no greater than 0.1 wt.% based on the aerosolisable formulation. In one aspect the one or more cyclodextrins are present in a total amount of no greater than 0.01 wt.% based on the aerosolisable formulation. In one aspect the one or more cyclodextrins are present in a total amount of no greater than 0.001 wt.% based on the aerosolisable formulation.

One or more cyclodextrins may or may not be present in any suitable amount in the aerosolised formulation. In one aspect the one or more cyclodextrins are present in a total amount of no greater than 12 wt.% based on the aerosolised formulation. In one aspect the one or more cyclodextrins are present in a total amount of no greater than 10 wt.% based on the aerosolised formulation. In one aspect the one or more cyclodextrins are present in a total amount of no greater than 9 wt.% based on the aerosolised formulation. In one aspect the one or more cyclodextrins are present in a total amount of no greater than 8 wt.% based on the aerosolised formulation. In one aspect the one or more cyclodextrins are present in a total amount of no greater than 7 wt.% based on the aerosolised formulation. In one aspect the one or more cyclodextrins are present in a total amount of no greater than 6 wt.% based on the aerosolised formulation. In one aspect the one or more cyclodextrins are present in a total amount of no greater than 5 wt.% based on the aerosolised formulation. In one aspect the one or more cyclodextrins are present in a total amount of no greater than 4 wt.% based on the aerosolised formulation. In one aspect the one or more cyclodextrins are present in a total amount of no greater than 3 wt.% based on the aerosolised formulation. In one aspect the one or more cyclodextrins are present in a total amount of no greater than 2 wt.% based on the aerosolised formulation. In one aspect the one or more cyclodextrins are present in a total amount of no greater than 1 wt.% based on the aerosolised formulation. In one aspect the one or more cyclodextrins are present in a total amount of no greater than 0.5 wt.% based on the aerosolised formulation. In one aspect the one or more cyclodextrins are present in a total amount of no greater than 0.1 wt.% based on the aerosolised formulation. In one aspect the one or more cyclodextrins are present in a total amount of no greater than 0.01 wt.% based on the aerosolised formulation. In one aspect the one or more cyclodextrins are present in a total amount of no greater than 0.001 wt.% based on the aerosolised formulation.

The one or more cyclodextrins may be selected from the group consisting of unsubstituted cyclodextrins, substituted cyclodextrins and mixtures thereof. In one aspect at least one cyclodextrin is an unsubstituted cyclodextrin. In one aspect the one or more cyclodextrins are selected from the group consisting of unsubstituted cyclodextrins. In one aspect at least one cyclodextrin is a substituted cyclodextrin. In one aspect the one or more cyclodextrins are selected from the group consisting of substituted cyclodextrins.

In one aspect the one or more cyclodextrins are selected from the group consisting of unsubstituted (α)-cyclodextrin, substituted (α)-cyclodextrin, unsubstituted (β)-cyclodextrin, substituted (β)-cyclodextrin, unsubstituted (γ)-cyclodextrin, substituted (γ)-cyclodextrin, and mixtures thereof. In one aspect the one or more cyclodextrins are selected from the group consisting of unsubstituted (β)-cyclodextrin, substituted (β)-cyclodextrin, and mixtures thereof.

In one aspect the one or more cyclodextrins are selected from the group consisting of unsubstituted (α)-cyclodextrin, unsubstituted (β)-cyclodextrin, unsubstituted (y)-cyclodextrin, and mixtures thereof. In one aspect the one or more cyclodextrins is selected from unsubstituted (β)-cyclodextrin.

In one aspect the one or more cyclodextrins are selected from the group consisting of substituted (α)-cyclodextrin, substituted (β)-cyclodextrin, substituted (γ)-cyclodextrin, and mixtures thereof. In one aspect the one or more cyclodextrins is selected from substituted (β)-cyclodextrins. Chemical substitutions at the 2-, 3-, and 6-hydroxyl sites are envisaged, and in particular substitution at the 2-position.

In one aspect the one or more cyclodextrins are selected from the group consisting of 2-hydroxy-propyl-α-cyclodextrin, 2-hydroxy-propyl-β-cyclodextrin, 2-hydroxy-propyl-γ-cyclodextrin and mixtures thereof. In one aspect the one or more cyclodextrins is at least 2-hydroxy-propyl-α-cyclodextrin. In one aspect the one or more cyclodextrins is at least 2-hydroxy-propyl-β-cyclodextrin. In one aspect the one or more cyclodextrins is at least 2-hydroxy-propyl-γ-cyclodextrin.

2-hydroxy-propyl derivatives of cyclodextrins, such as 2-hydroxy-propyl-β-cyclodextrin have increased solubility in water when compared to base cyclodextrins such as β-cyclodextrin.

In one aspect if the aerosolisable formulation contains one or more cyclodextrins, then the aerosolisable formulation contains no flavours that can be encapsulated by the one or more cyclodextrins.

### Process

As discussed herein, in one aspect there is provided a process for forming an aerosol, the process comprising aerosolising an aerosolisable formulation comprising
(i) water in an amount of at least 50 wt.% based on the aerosolisable formulation; and
(ii) at least one active agent; to provide an aerosol having the following particle size distribution: D50 of from 3 to 6µm and D10 of at least 0.5µm.

In the process the aerosol may be formed by a process performed at a temperature below 60°C.In the process the aerosol may be formed by a process performed at a temperature below 50°C. In the process the aerosol may be formed by a process performed at a temperature below 40°C. In the process the aerosol may be formed by a process performed at a temperature below 30°C. In the process the aerosol may be formed by a process performed at a temperature below 25°C. In the process the aerosol may be formed by a process which does not involve heating.

In the process the aerosol may be formed by applying ultrasonic energy to the aerosolisable formulation.

The formulation may be contained or delivered by any means. In one aspect the present invention provides a contained aerosolisable formulation comprising (a) one or more containers; and (b) an aerosolisable formulation as defined herein. The container may be any suitable container, for example to allow for the storage or delivery of the formulation. In one aspect the container is configured for engagement with an electronic aerosol provision system. The container may be configured to become fluidly in communication with an electronic aerosol provision system so that formulation may be delivered to the electronic aerosol provision system. As described above, the present disclosure relates to container which may be used in an electronic aerosol provision system, such as an e-cigarette. Throughout the following description the term "e-cigarette" is used; however, this term may be used interchangeably with electronic aerosol provision system.

As discussed herein, the container of the present invention is typically provided for the delivery of aerosolisable formulation to or within an e-cigarette. The aerosolisable formulation may be held within an e-cigarette or may be sold as a separate container for subsequent use with or in an e-cigarette. As understood by one skilled in the art, e-cigarettes may contain a unit known as a detachable cartomiser which typically comprises a reservoir of aerosolisable formulation, and an aerosoliser such as a wick material and a heating element for vaporising the aerosolisable formulation. In some e-cigarettes, the cartomiser is part of a single-piece device and is not detachable. In one aspect the container is a cartomiser or is part of a cartomiser. In one aspect the container is not a cartomiser or part of a cartomiser and is a container, such as a tank, which may be used to deliver nicotine formulation to or within an e-cigarette.

In one aspect the container is part of an e-cigarette. Therefore in a further aspect the present invention provides an electronic aerosol provision system comprising: an aerosolisable formulation as defined herein; an aerosoliser for aerosolising formulation for inhalation by a user of the electronic aerosol provision system; and a power supply comprising a cell or battery for supplying power to the aerosoliser.

In addition to the aerosolisable formulation of the present invention and to systems such as containers and electronic aerosol provision systems containing the same, the present invention provides a process for improving the sensory properties of an aerosolised nicotine. In a further aspect the present invention provides a process for improving the storage stability of an aerosolised nicotine formulation.

Reference to an improvement in the sensory properties of a vaporised nicotine solution refer may include an improvement in the smoothness of the vaporised nicotine solution as perceived by a user.

The process of the present invention may comprises additional steps either before the steps listed, after the steps listed or between one or more of the steps listed.

### BRIEF DESCRIPTION OF THE DRAWINGS

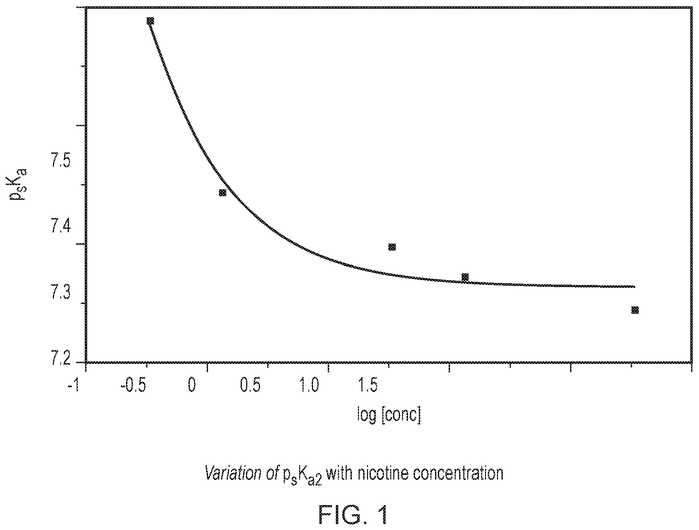

The present invention will now be described in further detail by way of example only with reference to the accompanying figure in which:-
Figure 1 shows a graph illustrating variation of pₛKₐ₂ with nicotine concentration; and

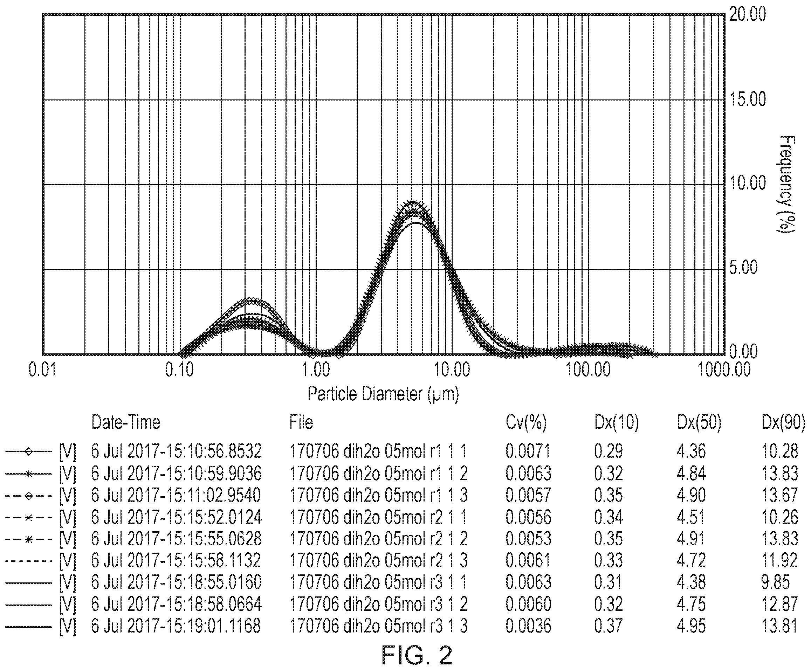

Figure 2 shows particle size distribution of the present aerosolised formulation.

The invention will now be described with reference to the following non-limiting example.

### Examples

A series of tests were conducted using a commercially available vibrating mesh nebuliser device. The device was loaded with formulation containing 90.0% (w/w) water, 9.0% 2-hydroxy-propyl-β-cyclodextrin, 0.9% I-menthol, and 0.1% nicotine.

The Droplet Size Distribution (DSD) formed on actuation of the device was determined primarily by laser diffraction. For example, the Malvern Instruments (Worcestershire, UK) Spraytec system uses these principles to determine the droplet size distribution of aerosols in the range from 0.1 - 900 µm.

The position of the aerosol source (e.g. the Device mouthpiece) was kept constant and at a fixed distance from the laser beam (2.5 cm) for the duration of the measurements, and to ensure appropriate actuation into the path of the laser beam. Data collection was triggered by the change in light transmittance as droplets entered the measurement zone and continued over 3 seconds at a sampling rate of 2.5 kHz.

Both the British Pharmacopoeia Commission (2014) [British Pharmacopoeia. London, England: Stationery Office] and The Council of Europe (2013) [European Pharmacopoeia. Strasbourg, France: Council of Europe] have published standards relating to the validity of Laser Diffraction procedures as a relevant test methodology for medicinal substances, including nicotine. Acceptance criteria for *x*₅₀ data (central distribution value) are specified as Sᵣₑₗ ≤ 10% over at least 6 replicates. Additionally, the acceptance criteria for *x*₁₀ and *x*₉₀ data (values at the sides of the distribution) are specified as Sᵣₑₗ ≤ 15% over at least 6 replicates. It should be noted that these conventions include the caveat that the criteria must be doubled for values below 10 µm in size.

It is recognised that Laser Diffraction techniques are more widely available that alternative techniques (e.g. cascade impactors) and have a longer history of use for product testing for inhaled aerosols.

Various modifications and variations of the present invention will be apparent to those skilled in the art without departing from the scope of the invention. Although the invention has been described in connection with specific preferred embodiments, it should be understood that the invention as claimed should not be unduly limited to such specific embodiments.

## Claims

1. An aerosolised formulation comprising
(i) water in an amount of at least 50 wt.% based on the aerosolised formulation; and
(ii) at least one active agent;
wherein the aerosol has the following particle size distribution: D50 of from 2 to 6µm, and
wherein the aerosol has a D10 of at least 0.5µm.

2. An aerosolised formulation according to claim 1 wherein water is present in an amount of at least 75 wt.% based on the aerosolised formulation, such as wherein water is present in an amount of at least 90 wt.% based on the aerosolised formulation.

3. An aerosolised formulation according to claim 1 or 2 wherein the active agent is present in an amount of no greater than 1 wt.% based on the aerosolised formulation, such as wherein the active agent is present in an amount of from 0.01 to 0.6 wt.% based on the aerosolised formulation, and optionally wherein the active agent is at least nicotine.

4. An aerosolised formulation according to any one of claims 1 to 3 wherein the aerosol has a D50 of from 3.5 to 5.5µm, such as wherein the aerosol has a D50 of from 4.5 to 5.5µm.

5. An aerosolised formulation according to any one of claims 1 to 4 wherein the aerosol has a D90 of no greater than 15µm.

6. An aerosolised formulation according to any one of claims 1 to 5 wherein the aerosolised formulation further comprises an acid, such as wherein the acid is selected from the group consisting of acetic acid, lactic acid, formic acid, citric acid, benzoic acid, pyruvic acid, levulinic acid, succinic acid, tartaric acid, sorbic acid, propionic acid, phenylacetic acid, and mixtures thereof, such as wherein the acid is selected from the group consisting of citric acid, benzoic acid, levulinic acid, sorbic acid, lactic acid, and mixtures thereof, such as wherein the acid is at least citric acid.

7. An aerosolised formulation according to claim 6 wherein the total content of acid present in the formulation is no greater than 1 mole equivalents based on the active agent, such as wherein the total content of acid present in the formulation is no less than 0.01 mole equivalents based on the active agent.

8. An aerosolised formulation according to any one of claims 1 to 7 further comprising one or more flavours, such as wherein the one or more flavours are selected from the group consisting of (4-(para-)methoxyphenyl)-2-butanone, vanillin, γ-undecalactone, menthone, 5-propenyl guaethol, menthol, para-mentha-8-thiol-3-one and mixtures thereof, such as wherein the flavour is at least menthol.

9. An aerosolised formulation according to claim 8 wherein the one or more flavours are present in a total amount of no greater than 2wt.% based on the aerosolised formulation, such as wherein the one or more flavours are present in a total amount of from 0.01 to 1wt.% based on the aerosolised formulation.

10. A process for forming an aerosol, the process comprising aerosolising an aerosolisable formulation comprising
(i) water in an amount of at least 50 wt.% based on the aerosolisable formulation; and
(ii) at least one active agent;
to provide an aerosol having the following particle size distribution: D50 of from 3 to 6µm and D10 of at least 0.5µm.

11. A process according to claim 10 wherein the aerosol is formed by a process performed at a temperature below 50°C such as wherein the aerosol is formed by applying ultrasonic energy to the aerosolised formulation.

12. An electronic aerosol provision system comprising:
(a) an aerosoliser for aerosolising formulation for inhalation by a user of the electronic aerosol provision system;
(b) a power supply comprising a cell or battery for supplying power to the aerosoliser
(c) aerosolisable formulation comprising
(i) water in an amount of at least 50 wt.% based on the aerosolisable formulation; and
(ii) at least one active agent;
wherein the aerosoliser provides an aerosol having the following particle size distribution: D50 of from 2 to 6µm and D10 of at least 0.5µm.

13. An electronic aerosol provision system according to claim 12 wherein the aerosolised formulation is an aerosolised formulation as defined in any one of claims 2 to 9.

14. A process for improving delivery of an aerosolised formulation to the lungs, the process comprising the steps of
(a) providing an aerosolisable material comprising (i) water in an amount of at least 50 wt.% based on the aerosolisable material and (ii) at least one active agent;
(b) aerosolising the material aerosolisable material to provide an aerosol having the following particle size distribution: D50 of from 2 to 6µm and D10 of at least 0.5µm.

## Patentansprüche

1. Aerosolisierte Formulierung, umfassend
(i) Wasser in einer Menge von mindestens 50 Gew.-%, bezogen auf die aerosolisierte Formulierung,
(ii) mindestens einen Wirkstoff;
wobei das Aerosol die folgende Partikelgrößenverteilung aufweist: D50 von 2 bis 6 µm, und
wobei das Aerosol einen D10 von mindestens 0,5 µm aufweist.

2. Aerosolisierte Formulierung nach Anspruch 1, wobei das Wasser in einer Menge von mindestens 75 Gew.-%, bezogen auf die aerosolisierte Formulierung, vorhanden ist, wobei Wasser beispielsweise in einer Menge von mindestens 90 Gew.-%, bezogen auf die aerosolisierte Formulierung, vorhanden ist.

3. Aerosolisierte Formulierung nach Anspruch 1 oder 2, wobei der Wirkstoff in einer Menge von nicht mehr als 1 Gew.-%, bezogen auf die aerosolisierte Formulierung, vorhanden ist, wobei der Wirkstoff beispielsweise in einer Menge von 0,01 bis 0,6 Gew.-%, bezogen auf die aerosolisierte Formulierung, vorhanden ist und wobei es sich bei dem Wirkstoff gegebenenfalls um mindestens Nicotin handelt.

4. Aerosolisierte Formulierung nach einem der Ansprüche 1 bis 3, wobei das Aerosol einen D50 von 3,5 bis 5,5 µm aufweist, wobei das Aerosol beispielsweise einen D50 von 4,5 bis 5,5 µm aufweist.

5. Aerosolisierte Formulierung nach einem der Ansprüche 1 bis 4, wobei das Aerosol einen D90 von höchstens 15 µm aufweist.

6. Aerosolisierte Formulierung nach einem der Ansprüche 1 bis 5, wobei die aerosolisierte Formulierung ferner eine Säure umfasst, wobei die Säure beispielsweise aus der Gruppe bestehend aus Essigsäure, Milchsäure, Ameisensäure, Citronensäure, Benzoesäure, Brenztraubensäure, Lävulinsäure, Bernsteinsäure, Weinsäure, Sorbinsäure, Propionsäure, Phenylessigsäure und Mischungen davon ausgewählt ist, wobei die Säure beispielsweise aus der Gruppe bestehend aus Citronensäure, Benzoesäure, Lävulinsäure, Sorbinsäure, Milchsäure, und Mischungen davon ausgewählt ist, wobei es sich bei der Säure beispielsweise um mindestens Citronensäure handelt.

7. Aerosolisierte Formulierung nach Anspruch 6, wobei der Gesamtgehalt an in der Formulierung vorhandener Säure höchstens 1 Moläquivalent bezogen auf den Wirkstoff beträgt, wobei der Gesamtgehalt an in der Formulierung vorhandener Säure beispielsweise nicht weniger als 0,01 Moläquivalente bezogen auf den Wirkstoff beträgt.

8. Aerosolisierte Formulierung nach einem der Ansprüche 1 bis 7, ferner umfassend:
ein oder mehrere Aromen, wobei die einen oder mehreren Aromen beispielsweise aus der Gruppe bestehend aus (4-(para-)Methoxyphenyl)-2-butanon, Vanillin, γ-Undecalacton, Menthon, 5-Propenylguaethol, Menthol, para-Mentha-8-thiol-3-on und Mischungen davon ausgewählt sind, wobei es sich bei dem Aroma um mindestens Menthol handelt.

9. Aerosolisierte Formulierung nach Anspruch 8, wobei die einen oder mehreren Aromen in einer Gesamtmenge von höchstens 2 Gew.-%, bezogen auf die aerosolisierte Formulierung, vorhanden sind, wobei die einen oder mehreren Aromen in einer Gesamtmenge von 0,01 bis 1 Gew.-%, bezogen auf die aerosolisierte Formulierung, vorhanden sind.

10. Verfahren zur Bildung eines Aerosols, wobei das Verfahren das Aerosolisieren einer aerosolisierbaren Formulierung umfasst, die Folgendes umfasst:
(i) Wasser in einer Menge von mindestens 50 Gew.-%, bezogen auf die aerosolisierbare Formulierung; und
(ii) mindestens einen Wirkstoff;
sodass ein Aerosol mit der folgenden Partikelgrößenverteilung bereitgestellt wird: D50 von 3 bis 6 µm und D10 von mindestens 0,5 µm.

11. Verfahren nach Anspruch 10, wobei das Aerosol durch ein bei einer Temperatur unter 50 °C durchgeführtes Verfahren gebildet wird, wobei das Aerosol beispielsweise durch Anwendung von Ultraschallenergie auf die aerosolisierbare Formulierung gebildet wird.

12. Elektronisches Aerosolbereitstellungssystem, umfassend:
(a) einen Vernebler für eine Aerosolformulierung zur Inhalation durch einen Benutzer des elektronischen Aerosolbereitstellungssystems;
(b) eine Stromversorgung, die eine Zelle oder eine Batterie zur Versorgung des Verneblers mit Strom umfasst;
(c) eine aerosolisierbare Formulierung, die Folgendes umfasst:
(i) Wasser in einer Menge von mindestens 50 Gew.-%, bezogen auf die aerosolisierbare Formulierung; und
(ii) mindestens einen Wirkstoff;
wobei der Vernebler ein Aerosol mit der folgenden Partikelgrößenverteilung bereitstellt: D50 von 2 bis 6 µm und D10 von mindestens 0,5 µm.

13. Elektronisches Aerosolbereitstellungssystem nach Anspruch 12, wobei es sich bei der aerosolisierten Formulierung um eine wie in einem der Ansprüche 2 bis 9 definierte aerosolisierte Formulierung handelt.

14. Verfahren zur Verbesserung der Zuführung einer aerosolisierten Formulierung an die Lunge, wobei das Verfahren die folgenden Schritte umfasst:
(a) Bereitstellen eines aerosolierbaren Materials, das (i) Wasser in einer Menge von mindestens 50 Gew.-%, bezogen auf das aerosolierbare Material und (ii) mindestens einen Wirkstoff, umfasst;
b) Aerosolisieren des aerosolierbaren Material, sodass ein Aerosol mit der folgenden Partikelgrößenverteilung bereitgestellt wird: D50 von 2 bis 6 µm und D10 von mindestens 0,5 µm.

## Revendications

1. Formulation en aérosol comprenant
(i) de l'eau en une quantité d'au moins 50 % en poids par rapport à la formulation en aérosol ; et
(ii) au moins un agent actif ;
dans lequel l'aérosol présente la distribution granulométrique suivante : D50 de 2 à 6 µm, et
dans lequel l'aérosol a un D10 d'au moins 0,5 µm.

2. Formulation en aérosol selon la revendication 1, dans laquelle l'eau est présente en une quantité d'au moins 75 % en poids par rapport à la formulation en aérosol, tel que dans laquelle l'eau est présente en une quantité d'au moins 90 % en poids par rapport à la formulation en aérosol.

3. Formulation en aérosol selon la revendication 1 ou 2, dans laquelle l'agent actif est présent en une quantité non supérieure à 1 % en poids par rapport à la formulation en aérosol, tel que dans laquelle l'agent actif est présent en une quantité de 0,01 à 0,6 % en poids par rapport à la formulation en aérosol, et facultativement dans laquelle l'agent actif est au moins de la nicotine.

4. Formulation en aérosol selon l'une quelconque des revendications 1 à 3, dans laquelle l'aérosol a un D50 de 3,5 à 5,5 pm, tel que dans laquelle l'aérosol a un D50 de 4,5 à 5,5 µm.

5. Formulation en aérosol selon l'une quelconque des revendications 1 à 4, dans laquelle l'aérosol a un D90 non supérieur à 15 µm.

6. Formulation en aérosol selon l'une quelconque des revendications 1 à 5, dans laquelle la formulation en aérosol comprend en outre un acide, tel que dans laquelle l'acide est choisi dans le groupe constitué par l'acide acétique, l'acide lactique, l'acide formique, l'acide citrique, l'acide benzoïque, l'acide pyruvique, l'acide lévulinique, l'acide succinique, l'acide tartrique, l'acide sorbique, l'acide propionique, l'acide phénylacétique, et leurs mélanges, tel que dans laquelle l'acide est choisi dans le groupe constitué par l'acide citrique, l'acide benzoïque, l'acide lévulinique, l'acide sorbique, l'acide lactique, et leurs mélanges, tel que dans laquelle l'acide est au moins l'acide citrique.

7. Formulation en aérosol selon la revendication 6, dans laquelle la teneur totale en acide présent dans la formulation n'est pas supérieure à 1 équivalent molaire par rapport à l'agent actif, tel que dans laquelle la teneur totale en acide présent dans la formulation n'est pas inférieure à 0,01 équivalent molaire par rapport à l'agent actif.

8. Formulation en aérosol selon l'une quelconque des revendications 1 à 7, comprenant en outre un ou plusieurs arômes, tel que dans laquelle le ou les arômes sont choisis dans le groupe constitué par la (4-(para-)méthoxyphényl)-2-butanone, la vanilline, la γ-undécalactone, la menthone, le 5-propénylguaéthol, le menthol, la para-mentha-8-thiol-3-one et leurs mélanges, tel que dans laquelle l'arôme est au moins le menthol.

9. Formulation en aérosol selon la revendication 8, dans laquelle le ou les arômes sont présents en une quantité totale non supérieure à 2 % en poids par rapport à la formulation en aérosol, tel que dans laquelle le ou les arômes sont présents en une quantité totale de 0,01 à 1 % en poids par rapport à la formulation en aérosol.

10. Procédé pour la formation d'un aérosol, le procédé comprenant l'aérosolisation d'une formulation aérosolisable comprenant
(i) de l'eau en une quantité d'au moins 50 % en poids par rapport à la formulation aérosolisable ;
(ii) au moins un agent actif ;
pour fournir un aérosol ayant la distribution granulométrique suivante : D50 de 3 à 6 µm et D10 d'au moins 0,5 µm.

11. Procédé selon la revendication 10, dans lequel l'aérosol est formé par un procédé réalisé à une température inférieure à 50 °C, tel que dans lequel l'aérosol est formé en appliquant de l'énergie ultrasonique à la formulation en aérosol.

12. Système électronique de fourniture d'aérosol comprenant :
(a) un aérosoliseur pour l'aérosolisation d'une formulation pour inhalation par un utilisateur du système électronique de fourniture d'aérosol ;
(b) une alimentation électrique comprenant une pile ou une batterie pour l'apport d'énergie électrique à l'aérosoliseur
(c) une formulation aérosolisable comprenant
(i) de l'eau en une quantité d'au moins 50 % en poids par rapport à la formulation aérosolisable ;
(ii) au moins un agent actif ;
dans lequel l'aérosoliseur fournit un aérosol présentant la distribution granulométrique suivante : D50 de 2 à 6 µm et D10 d'au moins 0,5 µm.

13. Système électronique de fourniture d'aérosol selon la revendication 12, dans lequel la formulation en aérosol est une formulation en aérosol telle que définie dans l'une quelconque des revendications 2 à 9.

14. Procédé pour améliorer l'administration d'une formulation en aérosol aux poumons, le procédé comprenant les étapes de
(a) fourniture d'un matériau aérosolisable comprenant (i) de l'eau en une quantité d'au moins 50 % en poids par rapport au matériau aérosolisable et (ii) au moins un agent actif ;
(b) aérosolisation du matériau aérosolisable pour fournir un aérosol présentant la distribution granulométrique suivante : D50 de 2 à 6 µm et D10 d'au moins 0,5 µm.
